Europäisches Patentamt

European Patent Office

Office européen des brevets

(19)

(11) Numéro de publication: **0 267 088**
**A1**

## (12) DEMANDE DE BREVET EUROPEEN

(21) Numéro de dépôt: **87402288.2**

(22) Date de dépôt: **13.10.87**

(51) Int. Cl.⁴: **A 61 N 1/36**

(30) Priorité: **13.10.86 FR 8614187**

(43) Date de publication de la demande:
**11.05.88 Bulletin 88/19**

(84) Etats contractants désignés:
**AT BE CH DE ES FR GB GR IT LI LU NL SE**

(71) Demandeur: **COMPAGNIE FINANCIERE SAINT-NICOLAS**
**Allée Frion**
**F-93340 Le Raincy (FR)**

(72) Inventeur: **Buffet Jacques**
**43, rue Marc Viéville**
**F-93250 Villemomble (FR)**

(74) Mandataire: **Derambure, Christian**
**BUGNION ASSOCIES 116, Boulevard Haussmann**
**F-75008 Paris (FR)**

Le titre de l'invention a été modifié (Directives relatives à l'examen pratiqué à l'OEB, A-III, 7.3)

(54) **Programmateur externe destiné à la commande d'un stimulateur cardiaque implantable et stimulateur cardiaque à paramètres réglables.**

(57) Procédé de réglage d'un stimulateur (1) cardiaque implantable en fonction de l'effort du patient porteur du stimulateur (1), au moyen d'un programmateur externe de commande (2) associé fonctionnellement au stimulateur (1), dans lequel on définit préalablement plusieurs programme-types de fonctionnement $P_i$ du stimulateur (1) correspondant chacun à un type d'effort du patient porteur et caractérisés par desvaleurs spécifiques v d'au moins un paramètre de fonctionnement p du stimulateur (1); et on commande par voie externe, grâce au programmateur de commande (2) le stimulateur (1) pour sélectionner le programme-type de fonctionnement $P_i$ désiré, cette sélection ayant pour effet que le stimulateur (1) fonctionne alors selon le programme-type sélectionné; de manière que le patient porteur puisse choisir de façon autonome un programme-type de fonctionnement $P_i$ de son stimulateur (1) en fonction d'un effort à accomplir.

Selon l'invention on stocke dans la mémoire d'un microprocesseur (3) du stimulateur (1), les programmes types $P_i$ et donc les valeurs spécifiques V des paramètres p, à savoir d'une part, la fréquence maximale de stimulation F et, d'autre part, la durée D nécessaire pour atteindre cette fréquence F.

EP 0 267 088 A1

**Description**

### PROCEDE DE REGLAGE D'UN STIMULATEUR CARDIAQUE IMPLANTABLE EN FONCTION DE L'EFFORT DU PATIENT PORTEUR DU STIMULATEUR, STIMULATEUR CARDIAQUE IMPLANTABLE A PARAMETRES REGLABLES ET PROGRAMMATEUR EXTERNE DE COMMANDE D'UN STIMULATEUR CARDIAQUE IMPLANTABLE REGLABLE.

L'invention concerne un procédé de réglage d'un stimulateur cardiaque implantable en fonction de l'effort du patient porteur du stimulateur, au moyen d'un programmateur externe de commande associé fonctionnellement au stimulateur; un stimulateur cardiaque implantable à paramètres réglables en fonction de l'effort du patient porteur; un programmateur externe de commande d'un stimulateur cardiaque implantable permettant le réglage de paramètres de fonctionnement du stimulateur, ce dernier comprenant des moyens récepteurs susceptibles d'être couplés au programmateur.

On connaît déjà des stimulateurs cardiaques réglables (brevets français 2 379 104, 2 383 674, 2 471 789, 2 431 296, 2 424 737, 2 342 722, 2 374 024, 2 550 095, 2 516 797, brevet européen 96 464, brevet américain 3 867 950) qui sont de l'un des deux types suivants : à commande externe ou à commande interne, ces deux types n'étant pas exclusifs l'un de l'autre.

La commande externe connue est réalisée à partir d'un programmateur externe, en général par le médecin, et vise à adapter, lorsque nécessaire, mais le plus souvent occasionnellement, les différents paramètres du stimulateur (période de stimulation, largeur de l'impulsion de stimulation, amplitude de l'impulsion de stimulation, sensibilité de l'amplificateur du stimulateur, période réfractaire, période d'échappement, etc ...). Dans ce premier cas, le programmateur et le stimulateur sont agencés pour que les paramètres puissent être réglés séparément et successivement. Ce réglage nécessite un certain temps et un contrôle de la part du médecin, ce qui n'est pas en soi gênant, dès lors que le réglage reste occasionnel. Un exemple typique de réalisation d'un tel stimulateur est décrit dans le brevet français 2 424 737. Un autre exemple typique de réalisation est le stimulateur à deux vitesses commutables; un champ magnétique créé par un aimant puissant placé en regard du site d'implantation du stimulateur permet de le faire basculer d'une première vitesse de fonctionnement à une seconde vitesse de fonctionnement et donc d'adapter le stimulateur aux besoins. Toutefois, cette réalisation, bien qu'ayant l'avantage de la simplicité a l'inconvénient d'être de portée limitée.

La commande interne connue est réalisée en général automatiquement en fonction de l'état du patient porteur du stimulateur, grâce à un paramètre représentatif de cet état. Différents exemples typiques de réalisation sont connus : stimulateur piloté par l'activité auriculaire ou la température dans le flux veineux ou la fréquence respiratoire ou la distance entre l'onde R et le sommet de l'onde T de l'activité cardiaque, etc ... Ces réalisations ont l'avantage de l'automaticité du réglage, mais posent de nombreux problèmes : manque de fiabilité du maintien en place d'une électrode auriculaire de recueil de l'activité électrique auriculaire, corrélation mal établie entre le paramètre mesuré et les valeurs données au paramètre de fonctionnement du stimulateur, difficultés de détermination avec précision de la position de l'onde T, etc... en fonction du paramètre considéré.

On connaît aussi (document FR 2481933) un programmateur de stimulateur cardiaque pouvant être manoeuvré par le patient soi-même, ce programmateur comportant un curseur permettant de sélectionner une fréquence de fonctionnement du stimulateur cardiaque parmi trois, plus précisément la fréquence correspondant à un certain besoin physiologique. Toutefois, un tel programmateur présente certains inconvénients : le passage d'une fréquence à une autre intervient brutalement, le patient risque d'oublier à quelle fréquence fonctionne son stimulateur, les programmateurs sont spécifiques et non interchangeables, les modes de fonctionnement du stimulateur sont peu élaborés, le programmateur peut être violé et modifié, la mise en oeuvre du programmateur implique sa disposition en regard du stimulateur ce qui n'est pas commode, bref il découle un certain danger de mise en oeuvre, un coût important du matériel, un caractère peu sophistiqué.

L'invention a pour but de proposer un stimulateur réglable par voie externe en fonction des efforts du patient qui soit à la fois simple de construction et de fonctionnement, mais permette un réglage assez large des paramètres.

Un autre but est de proposer un tel stimulateur qui soit autoréglable, c'est-à-dire réglable par le patient porteur lui-même.

A cet effet, l'invention propose d'abord un procédé de réglage d'un stimulateur cardiaque implantable en fonction de l'effort du patient porteur du stimulateur, au moyen d'un programmateur externe de commande associé fonctionnellement au stimulateur dans lequel on définit préalablement plusieurs programme-types de fonctionnement du stimulateur correspondant chacun à un type d'effort du patient porteur, et caractérisés par des valeurs spécifiques d'au moins un paramètre de fonctionnement du stimulateur; et on commande par voie externe, grâce au programmateur de commande, le stimulateur pour sélectionner le programme-type de fonctionnement désiré, cette sélection ayant pour effet que le stimulateur fonctionne alors selon le programme-type sélectionné; de manière que le patient porteur puisse choisir de façon autonome un programme-type de fonctionnement de son stimulateur en fonction d'un effort à accomplir, caractérisé en ce que l'on stocke dans la mémoire d'un microprocesseur du stimulateur, les programmes types et donc la valeur spécifique des paramètres, à savoir d'une part, la fréquence maximale de stimulation et, d'autre part, la durée D nécessaire pour atteindre cette fréquence.

L'invention propose ensuite un stimulateur cardiaque implantable à paramètres réglables en fonction de l'effort du patient porteur grâce à des moyens de sélection d'un programme type de fonctionnement, par voie externe caractérisé par le fait que le stimulateur comporte un microprocesseur dans la mémoire duquel sont stockés plusieurs programme-types de fonctionnement, caractérisés par des valeurs spécifiques d'au moins un paramètre de fonctionnement du stimulateur.

L'invention propose enfin un programmateur externe de commande d'un stimulateur cardiaque implantable permettant le réglage des paramètres de fonctionnement du stimulateur, ce dernier comprenant des moyens récepteurs susceptibles d'être couplés au programmateur, caractérisé par le fait qu'il comporte au moins un sélecteur de programme-type , parmi plusieurs types caractérisés par des valeurs spécifiques d'au moins un paramètre du stimulateur, à savoir un boîtier, une source d'énergie électrique, telle qu'une pile logée dans le boîtier, des contacts électriques de transmission d'impulsions électriques au stimulateur cardiaque en vue de sa commande ; un circuit électronique associé à la source d'énergie électrique, aux contacts, au sélecteur ayant pour fonction de délivrer aux contacts des trains d'impulsion spécifiques en fonction de l'état du sélecteur.

Selon une autre caractéristique de l'invention, on peut également régler, par voie externe, grâce à un autre programmateur externe de réglage, les valeurs spécifiques des paramètres de chaque programme-type de manière à adapter les programme-type aux besoins du patient porteur.

L'invention a pour avantage de permettre au patient porteur de régler lui-même le programme de fonctionnement de son stimulateur et ceci à partir de plusieurs programme-type, correspondant à des catégories homogènes d'effort.

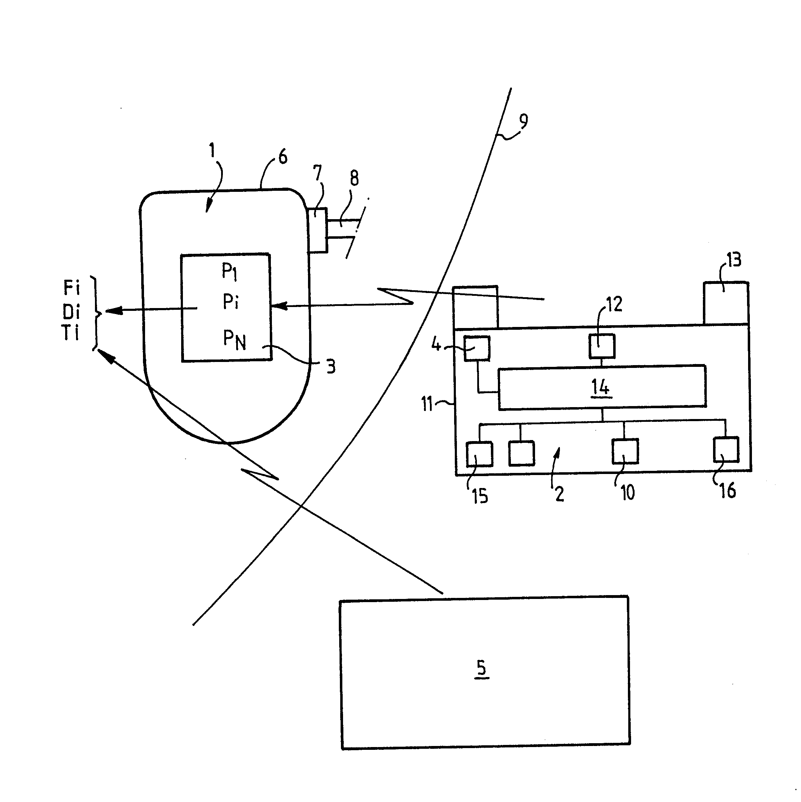

Les autres caractéristiques de l'invention résulteront de la description qui suivra en référence à la figure unique annexée qui illustre l'invention.

L'invention concerne d'abord un procédé de réglage d'un stimulateur cardiaque implantable 1 en fonction de l'effort du patient porteur de ce stimulateur 1, au moyen d'un programmateur externe de commande 2, associé fonctionnellement au stimulateur 1. L'invention concerne ensuite le stimulateur cardiaque implantable 1 dont les paramètres sont réglables en fonction de l'effort du patient porteur. L'invention concerne enfin le programmateur externe de commande 2 du stimulateur cardiaque 1, ce programmateur permettant le réglage de paramètres de fonctionnement du stimulateur 1.

Dans le procédé selon l'invention, on définit préalablement plusieurs programme-type de fonctionnement $P_I$... $P_i$ ... $P_n$ du stimulateur 1. Chaque programme-type $P_I$ correspond à un type d'effort du patient porteur. Par exemple, un premier programme-type correspond à une marche à l'allure de promenade, un deuxième programme à la montée d'un escalier, un troisième programme à tel ou tel sport. Chaque programme-type est caractérisé par des valeurs spécifiques v d'au moins un paramètre de fonctionnement p du stimulateur 1.

On stocke dans la mémoire d'un microprocesseur 3 du stimulateur 1 les différents programme-type $P_i$ et donc, les valeurs spécifiques correspondantes v des paramètres p.

Puis, on commande par voie externe, grâce au programmateur de commande 2, le microprocesseur 3 pour sélectionner le programme-type de fonctionnement désiré $P_i$, cette sélection ayant pour effet que le stimulateur 1 fonctionne alors selon le programme-type sélectionné $P_i$.

Il en résulte que le patient porteur du stimulateur 1 peut choisir de façon autonome un programme-type de fonctionnement $P_i$ de son stimulateur en fonction d'un effort à accomplir, ramené à un effort-type correspondant au programme type.

Selon une autre caractéristique importante de l'invention, pour chaque programme-type $P_i$ de fonctionnement du stimulateur 1, on définit comme paramètre p d'une part, la fréquence maximale de stimulation F et d'autre part, la durée D nécessaire pour atteindre cette fréquence. Naturellement l'invention s'applique aussi au cas où d'autres paramètres (différents ou/et en plus) sont réglés.

Egalement, préférentiellement, pour chaque programme-type $P_i$, on limite la durée de fonctionnement du stimulateur à la fréquence maximale F à une durée spécifique prédéterminée T. A cet effet, on stocke les durées spécifiques T des programme-type $P_i$ dans la mémoire du microprocesseur 3 (ou, en variante dans le programmateur 2).

Ces deux caractéristiques ont pour effet d'éviter le changement brutal et dangereux de la fréquence du stimulateur ou le maintien excessif à une telle fréquence.

Ainsi, pour chaque programme $P_I$, sont définies, par exemple, trois valeurs spécifiques respectivement $F_I$, $D_I$ et $T_I$, correspondant respectivement à une fréquence maximale de stimulation, à une durée nécessaire pour atteindre cette fréquence maximale et enfin à une durée de fonctionnement du stimulateur à cette fréquence maximale. De plus, on stocke dans la mémoire du microprocesseur 3 plusieurs programmes $P_I$ ... $P_i$... $P_n$. Pour des raisons de simplicité de fonctionnement et pour éviter que la mémoire du microprocesseur 3 ne soit trop importante, le nombre n de programmes stockés est limité à quelques programmes, par exemple, à un nombre compris entre 3 et 10 programmes. Toutefois, l'invention s'applique également à un nombre de programmes stockés beaucoup plus important.

La caractéristique même de chacun des programme-type $P_i$ est une question de choix laissée au patient porteur et au médecin en fonction des nécessités. L'invention présente d'ailleurs comme avantage de permettre une grande souplesse de réglage selon chaque cas d'espèces, notamment les caractéristiques physiologiques du patient porteur, son activité, etc ... Les valeurs données aux paramètres F, D, T de chaque programme-type P sont également une question de choix faite par le médecin.

Selon une autre caractéristique de l'invention, on émet, grâce à un avertisseur 4 un signal lumineux et/ou sonore lorsque la durée de fonctionnement spécifique du stimulateur 1 selon un programme-type $P_i$ sélectionné est atteinte. Ce signal permet au patient porteur d'être informé de la fin du programme-type surtout dans le cas où, comme indiqué précédemment, la durée de fonctionnement du stimulateur à la fréquence maximale est prédéterminée, donc limitée.

Au lieu de fixer comme paramètre la durée de fonctionnement du stimulateur à la fréquence maximale d'un programme-type, on peut fixer comme paramètre la durée totale du programme-type lui-même.

La fixation d'une durée maximale de fonctionnement du stimulateur à la fréquence maximale combinée à la présence d'un signal d'avertissement de fin de programme constitue une mesure de sécurité permettant d'informer le patient porteur de la fin du programme-type, de limiter la durée pendant laquelle le stimulateur fonctionne à une fréquence élévée et enfin d'obliger le patient porteur dans le cas où l'effort se prolonge de relancer le programme-type.

Préférentiellement, on définit un ou plusieurs programmes transitoires de retour de fonctionnement du stimulateur mis en oeuvre automatiquement à la fin de chaque programme-type $P_i$ ou plus généralement, un protocole de retour du stimulateur 1 aux conditions normales de fonctionnement. Ce retour intervient progressivement pour des raisons de sécurité.

Selon une variante de réalisation préférentielle de l'invention, on règle également, par voie externe et grâce à un programmateur externe de réglage 5 les valeurs spécifiques v des paramètres P de chaque programme-type P, de manière à adapter les programme-type $P_i$ aux besoins du patient porteur. Dans ce cas, le procédé est organisé de manière à appeler successivement chaque programme et, pour chaque programme, chacun des paramètres du programme.

Un stimulateur cardiaque 1 selon l'invention est de type multiprogrammable, connu en soi, comportant un boîtier externe 6, une sortie 7 de connection électrique pour une électrode 8 (représentée partiellement). Dans le boîtier 6 sont logés une pile électrique, telle qu'une pile au lithium, un circuit électronique de commande associé à la pile, ainsi qu'à la sortie 7 et également, un microprocesseur 3 pour la mise en oeuvre du procédé.

Le boîtier 6, la sortie de connection électrique 7, l'électrode 8, la pile et le circuit électronique du stimulateur 1 ne font pas en soi directement l'objet de l'invention et sont généralement bien connus de l'homme de métier dans le cas d'un stimulateur de type multiprogrammable. Pour cette raison, ces différents éléments constitutifs ne sont pas décrits ici plus en détail.

Dans la mémoire du microprocesseur 3 sont stockés plusieurs programme-type $P_i$ de fonctionnement caractérisés par des valeurs spécifiques v d'au moins un paramètre de fonctionnement p du stimulateur. Le stimulateur 1 comporte également des moyens de sélection d'un programme-type $P_i$ de fonctionnement, donc, des valeurs spécifiques correspondantes des paramètres p, cette sélection étant opérée par voie externe, c'est-à-dire à travers la barrière cutanée 9 du patient porteur.

Comme indiqué précédemment, les paramètres de fonctionnement p dont les valeurs spécifiques v sont stockées dans la mémoire du microprocesseur 3 sont la fréquence maximale de stimulation F ou/et la durée D nécessaire pour atteindre cette fréquence F ou/et la dure de fonctionnement T du stimulateur à cette fréquence maximale.

De plus, est également stocké dans la mémoire du microprocesseur 3, un programme transitoire de retour du fonctionnement du stimulateur 1 aux conditions normales, ce programme étant mis en oeuvre automatiquement à la fin de chaque programme-type $P_i$.

Les moyens de sélection comportent des moyens récepteurs d'instructions de commande associées au microprocesseur 3 et susceptible d'être couplés au programmateur externe de commande 2. De plus, les mêmes ou d'autres moyens récepteurs d'instruction de réglage sont préférentiellement également associés au microprocesseur 3 et susceptibles d'être couplés à un programmateur externe de réglage 5.

Il est à noter que le réglage d'un paramètre d'un stimulateur cardiaque multiprogrammable par voie externe est en soi connu et l'invention met donc en oeuvre ces moyens connus. Par exemple, les moyens récepteurs peuvent être une antenne recevant des signaux redioélectriques ou encore un interrupteur REED recevant des champs magnétiques pulsés ou encore l'électrode de stimulation 8 recevant des courants galvaniques.

Une forme de réalisation structurelle possible d'un tel stimulateur cardiaque programmable est décrite par exemple dans le brevet français 2 424 737. Par exemple, la mémoire RAM du microprocesseur 3 est structurée en registres correspondant à la combinaison de groupes correspondant au nombre de programme-type et au nombre de paramètres réglables dans les programme-type.

En pratique, les valeurs v des paramètres p peuvent varier dans d'assez large limites. Par exemple, la fréquence peut varier entre 40 et 160 coups par minute et les durées D et T varier entre quelques minutes et une heure environ.

Le programmateur externe de commande 2 comporte au moins un sélecteur 10 du programme-type parmi plusieurs programmes-types, caractérisés par les valeurs spécifiques v d'au moins un paramètre p de fonctionnement du stimulateur 1 et ceci ainsi qu'il a été indiqué précédemment.

Dans une forme de réalisation possible, le programmateur de commande 2 comporte un boîtier 11, une source d'énergie électrique 12 telle qu'une pile logée dans le boîtier 11; des contacts électriques 13 émergeant du boîtier 11 et permettant la transmission d'impulsions électriques au stimulateur cardiaque en vue de sa commande; un circuit électronique 14 également logé dans le boîtier 11, associé à la source d'énergie électrique 12 et aux contacts 13, ainsi qu'au sélecteur 10. Ce circuit électronique 14 a pour fonction de délivrer aux contacts 13 des trains d'impulsion spécifiques en fonction de l'état du sélecteur 10.

Le sélecteur 10 peut être constitué par exemple, par une série de touches, à savoir, une touche "marche-arrêt" 15 et des touches 16 correspondant chacune à un programme-type.

Le fonctionnement d'un tel programmateur 2 peut être le suivant : lorsque le programmateur 2 est mis en marche, par actionnement de la touche 15, un premier train d'impulsion est émis grâce au circuit 14 et transmis au stimulateur 1 via les contacts 13 et les moyens de réception du stimulateur. Ce premier train d'impulsion est par exemple tel que sa longueur soit supérieure à toute période réfractaire programmable du stimulateur 1. Dès qu'il détecte un tel train d'impulsion, le stimulateur cardiaque 1 se règle automatiquement sur le mode de fonctionnement dit VVT dans lequel un signal est émis en synchronisme avec tout signal détecté. Puis, un train d'impulsion est émis ou non après chaque impulsion et cette présence ou cette absence du train d'impulsion correspond à une information binaire 0 ou 1 transmise au microprocesseur 3 et permettant le réglage de celui-ci. Pour des raisons de sécurité, on établit une redondance des signaux émis par le programmateur 2.

Le programmateur 2 peut comporter également un avertisseur 4 associé fonctionnellement au circuit électronique 14, ainsi qu'à une logique de fonctionnement du programmateur 2 en vue de l'émission d'un signal lumineux ou/sonore à la fin de chaque programme-type ou éventuellement, préventivement, avant la fin de ce programme.

Naturellement, le programmateur 2 peut faire l'objet de nombreuses variantes de réalisation en fonction notamment des moyens de communication avec le stimulateur 1 utilisé.

Enfin, l'invention peut mettre en oeuvre également un programmateur de réglage, 5, connu en soi, dont l'application est originale en ce qu'il permet de régler les valeurs de différents paramètres p et ceci en relation avec des programme-types $P_i$.

En ce qui concerne l'arrêt du programme-type sélectionné, deux cas sont envisageables : soit l'arrêt est volontaire, par le patient porteur lui-même grâce à la touche marche-arrêt 15, soit l'arrêt est automatique, du fait de la durée de fonctionnement maximale T préprogrammée. Dans ce dernier cas, deux variantes de réalisation sont possibles : soit la durée T est stockée dans la mémoire du microprocesseur (3), soit elle est stockée dans la logique de fonctionnement du programmateur 2.

**Revendications**

1) Procédé de réglage d'un stimulateur (1) cardiaque implantable en fonction de l'effort du patient porteur du stimulateur (1), au moyen d'un programmateur externe de commande (2) associé fonctionnellement au stimulateur (1), dans lequel on définit préalablement plusieurs programme-types de fonctionnement $P_i$ du stimulateur (1) correspondant chacun à un type d'effort du patient porteur et caractérisés par des valeurs spécifiques v d'au moins un paramètre de fonctionnement p du stimulateur (1); et on commande par voie externe, grâce au programmateur de commande (2) le stimulateur (1) pour sélectionner le programme-type de fonctionnement $P_i$ désiré, cette sélection ayant pour effet que le stimulateur (1) fonctionne alors selon le programme-type sélectionné; de manière que le patient porteur puisse choisir de façon autonome un programme-type de fonctionnement $P_i$ de son stimulateur (1) en fonction d'un effort à accomplir, caractérisé en ce que l'on stocke dans la mémoire d'un microprocesseur (3) du stimulateur (1), les programmes types $P_i$ et donc la valeur spécifique V des paramètres p, à savoir d'une part, la fréquence maximale de stimulation F et, d'autre part, la durée D nécessaire pour atteindre cette fréquence F.

2) Procédé selon la revendication 1, caractérisé par le fait que pour chaque programme-type $P_i$, on limite la durée de fonctionnement du stimulateur (1) à la fréquence maximale F à une durée spécifique déterminée T et, à cet effet, on stocke les durées spécifiques des programme-type P dans la mémoire du microprocesseur (3).

3) Procédé selon l'une quelconque des revendications 1 et 2, caractérisé par le fait qu'on émet, grâce à un avertisseur (4) un signal lumineux et/ou sonore lorsque la durée de fonctionnement spécifique du stimulateur (1) selon un programme-type sélectionné est atteinte.

4) Procédé selon l'une quelconque des revendications 1 à 3, caractérisé par le fait qu'on définit un programme transitoire de retour du fonctionnement du stimulateur (1) et on met en oeuvre automatiquement ce programme transitoire de retour à la fin de chaque programme-type P.

5) Procédé selon l'une quelconque des revendications 1 à 4, caractérisé par le fait qu'on règle, par voie externe, grâce à un programmateur externe de réglage (5) les valeurs spécifiques v des paramètres p de chaque programme de type P, de manière à adapter le programme-type P aux besoins du patient porteur.

6) Stimulateur cardiaque implantable à paramètres règlables en fonction de l'effort du patient porteur grâce à des moyens de sélection d'un programme type de fonctionnement P, par voie externe, caractérisé par le fait que le stimulateur (1) comporte un microprocesseur (3) dans la mémoire duquel sont stockés plusieurs programmes-types de fonctionnement P, caractérisés par des valeurs spécifiques v d'au moins un paramètre de fonctionnement p du stimulateur (1).

7) Stimulateur selon la revendication 6, caractérisé par le fait que les paramètres de fonctionnement p dont les valeurs spécifiques v sont stockées dans la mémoire du microprocesseur (3) sont la fréquence maximale de stimulation F, ou/et la durée D nécessaire pour atteindre cette fréquence F, ou/et la durée de fonctionnement T du stimulateur (1) à cette fréquence maximale F.

8) Stimulateur selon l'une quelconque des revendications 6 et 7, caractérisé par le fait qu'il comporte également, stocké dans la mémoire du microprocesseur (3), un programme transitoire de retour de fonctionnement du stimulateur (1) mis en oeuvre automatiquement à la fin de chaque programme-type P.

9) Stimulateur selon l'une quelconque des revendications 6 à 8, caractérisé par le fait qu'il comporte des moyens récepteurs d'instructions de commande associés au microprocesseur (3) et susceptibles d'être couplés à un programmateur externe de commande (2).

10) Stimulateur cardiaque selon l'une quelconque des revendications 6 à 9, caractérisé par le fait qu'il comporte des moyens récepteurs d'instructions de réglage associés au microprocesseur (3) et susceptibles d'être couplés à un programmateur externe de réglage (5).

11) Programmateur externe de commande d'un stimulateur cardiaque implantable (1) permettant le réglage de paramètres de fonctionnement p du stimulateur (1), ce dernier comprenant des moyens récepteurs susceptibles d'être couplés au programmateur (2), caractérisé par le fait qu'il comporte au moins un sélecteur de programme-type (10) parmi plusieurs types $P_l$ caractérisés par des valeurs spécifiques v d'au moins un paramètre p du stimulateur (1), à savoir un boîtier (11), une source d'énergie électrique (12), telle qu'une pile logée dans le boîtier (11), des contacts électriques (13) de transmission d'impulsions électriques au stimulateur cardiaque (1) en vue de sa commande; un circuit électronique (14) associé à la source d'énergie électrique (12), aux contacts (13), au sélecteur (10) ayant pour fonction de délivrer aux contacts (13) des trains d'impulsion spécifiques en fonction de l'état du sélecteur.

0267088

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| X | FR-A-2 481 933  (TELECTRONICS PTY. LTD) <br> * Figures 1-3,5-7; page 1, ligne 1 - page 3, ligne 7; page 6, lignes 2-33; page 7, ligne 27 - page 10, ligne 30; page 24, ligne 33 - page 27, ligne 31 * | 6 | A 61 N    1/36 |
| Y <br> A | | 11 <br> 1,5,7,9 ,10 | |
| D,Y | EP-A-0 005 392  (CARDIOFRANCE COMPAGNIE FRANCAISE D'ELECTRODARDIOLOGIE) <br> * Figures; page 3, ligne 36 - page 6, ligne 26; page 11, lignes 1-37 * | 11 | |
| D,A | | 1,5,6,9 ,10 | |
| D,A | FR-A-2 379 104  (PACESETTER SYSTEMS, INC.) <br> * Figures 1-3a,6,17,18; page 4, ligne 15 - page 5, ligne 24; page 10, ligne 13 - page 17, ligne 22; page 19, ligne 26 - page 20, ligne 31; page 27, ligne 20 - page 28, ligne 38; page 43, ligne 37 - page 47, ligne 12; page 50, ligne 6 - page 52, ligne 17 * | 1,5-7,9 -11 | |
| A | US-A-3 518 997  (SESSIONS) <br> * Figures 1,2; colonne 2, ligne 6 - colonne 3, ligne 46 * | 1 | DOMAINES TECHNIQUES RECHERCHES (Int. Cl.4) <br><br> A 61 N |

Le présent rapport a été établi pour toutes les revendications

| Lieu de la recherche | Date d'achèvement de la recherche | Examinateur |
|---|---|---|
| LA HAYE | 23-12-1987 | CHEN A.H. |